# EUROPEAN PATENT APPLICATION

(11) **EP 4 520 310 A1**
(43) Date of publication of application: **12.03.2025**
(21) Application number: 23816084.0
(22) Date of filing: 31.05.2023
(51) Int. Cl.: A61J 1/20, A61J 1/05, A61J 1/10, A61M 1/02, C12M 1/26, G01N 33/48

(54) **COLLECTION KIT AND COLLECTION METHOD**

(30) Priority: 31.05.2022 JP 2022088611
(71) Applicant: Terumo Kabushiki Kaisha, Tokyo 151-0072 (JP)
(72) Inventor: KATAGIRI, Mayu, Fujinomiya-shi, Shizuoka 418-0004 (JP); OKABE, Hiroshi, Fujinomiya-shi, Shizuoka 418-0004 (JP)
(74) Representative: TBK
(86) International application number: PCT/JP2023/020204
(87) International publication number: WO 2023/234327

(57) **Abstract**

A collection kit (10) includes: an inlet tube (12) to which a medical bag (94) accommodating an object to be collected is connected; and a collection bag (14) that is connected to a downstream side of the inlet tube (12), has a plurality of accommodation chambers (26, 28) formed by overlapping and welding two flexible sheets (22), and accommodates a predetermined amount of the object to be collected in each of the accommodation chambers (26, 28). The collection bag (14) integrally includes a tubular connection flow path (30) connecting the adjacent accommodation chambers (26, 28).

## Description

### Technical Field

The present invention relates to a collection kit and a collection method for collecting a blood product for inspection.

### Background Art

Blood products include a red blood cell product, a plasma product, a platelet product, and a whole blood product. As the blood products, products containing components required by patients are used for blood transfusion. The blood products are stored and transported in the state of being accommodated in medical bags. In order to ensure safety of the blood products, a small amount of sample may be collected to perform a culture test. In the culture test, the sample is collected in a culture bottle, and the culture bottle is placed in an environment suitable for bacterial growth to detect the presence or absence of a pathogen.

Among the blood products, a test for the platelet product is performed by the following procedure. A collection bag having a small volume is connected to a medical bag accommodating the platelet product (hereinafter, referred to as a platelet bag). Thereafter, a part of the platelet product in the platelet bag is transferred to the collection bag. Next, the collection bag is disconnected from the platelet bag.

Next, the collection bag is conveyed to a clean bench. In the clean bench, the platelet product in the collection bag is infused into a blood culture bottle. Prior to the infusion, a tube of the collection bag is connected to a tube extending from a sample collection tube. A prescribed amount of the platelet product is transferred from the collection bag to the sample collection tube with reference to scales of the sample collection tube.

Thereafter, an operator operates a valve of the sample collection tube while visually observing the scales of the sample collection tube to transfer the prescribed amount of the platelet product from the sample collection tube to the blood culture bottle. In the culture test, anaerobic culture and aerobic culture are performed. Therefore, the platelet product is dispensed into a culture bottle used for the anaerobic culture and a culture bottle used for the aerobic culture. Thereafter, the culture bottles are set in a culturing apparatus and subjected to the culture test for a predetermined period.

For example, US 8777921 discloses a collection device for collecting a sample from a medical bag.

### Summary of Invention

From the viewpoint of further improving the safety of a platelet product, a culture test for a total number of the platelet product has been studied. Therefore, it is required to improve the work efficiency of the culture test of the platelet product. In addition, in the case of performing the culture test for the total number, it is desirable that an instrument for distributing the platelet product into culture bottles has a simple structure such that cost can be reduced.

An object of the present invention is to solve the problem.

According to one aspect of the following disclosure, there is provided a collection kit including: an inlet tube to which a medical bag accommodating an object to be collected is connected; a collection bag which is connected to a downstream side of the inlet tube, includes a plurality of accommodation chambers formed by overlapping and welding two flexible sheets, and accommodates a predetermined amount of the object to be collected in each of the accommodation chambers; a plurality of outlet ports which are connected to the accommodation chambers, respectively, and allow the object to be collected to flow out from the accommodation chambers; and adapters which are connected to downstream sides of the outlet ports, respectively, and connect culture bottles and the outlet ports by the culture bottles being attached to the adapters, respectively, wherein the collection bag includes a connection flow path connecting the accommodation chambers adjacent to each other, and the connection flow path is integrally formed with the accommodation chambers by the two flexible sheets, and has a tubular portion formed in a tubular shape by cutting out the flexible sheets around the tubular portion between the accommodation chambers adjacent to each other.

In addition, according to another aspect, there is provided a collection method using the collection kit of the above aspect, the collection method including: a step of connecting the medical bag to the inlet tube; a step of introducing the object to be collected into the accommodation chambers of the collection bag through the inlet tube; a step of separating the medical bag from the inlet tube; a step of closing the tubular portion to prevent the object to be collected from moving between the plurality of accommodation chambers; and a step of connecting the culture bottles to the adapters and injecting the object to be collected into the culture bottles.

The collection kit and the collection method from the above aspects have a simple configuration, and thus can be manufactured at low cost and have excellent work efficiency.

### Brief Description of Drawings

Fig. 1 is a plan view of a collection kit according to a first embodiment.
Fig. 2 is an explanatory view of a step of introducing a platelet product into the collection kit of Fig. 1.
Fig. 3 is an explanatory view of a step of introducing the platelet product introduced into first and second accommodation chambers of Fig. 1 into the first and second culture bottles.
Fig. 4 is a schematic explanatory view of a collection kit according to a second embodiment.
Fig. 5A is a perspective view of the collection kit of Fig. 4, and Fig. 5B is a cross-sectional view taken along line VB-VB of Fig. 4.

### Description of Embodiments

### (First Embodiment)

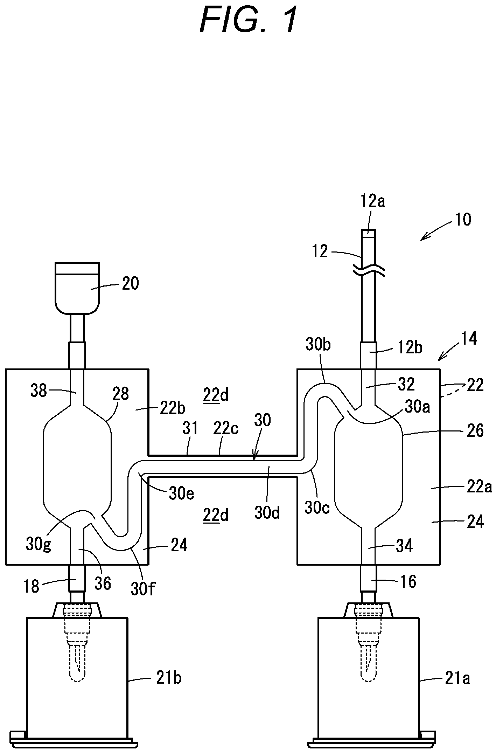

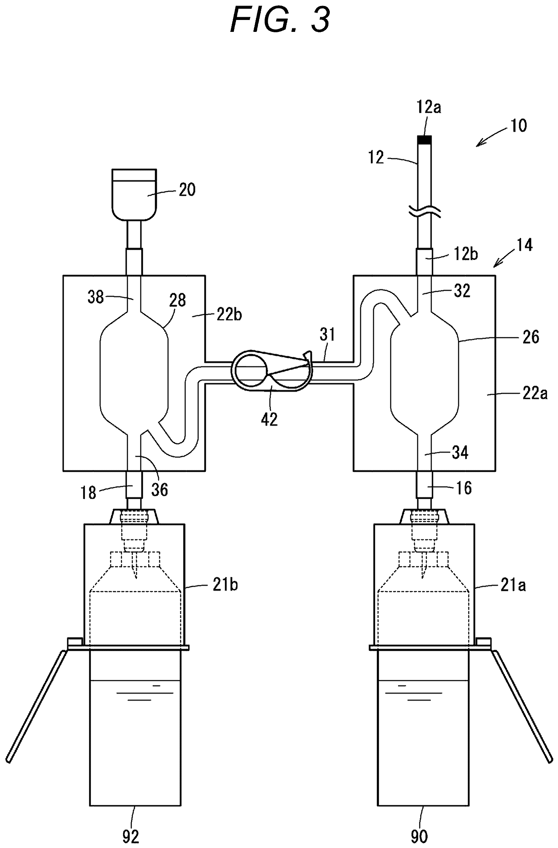

A collection kit 10 according to the present embodiment illustrated in Fig. 1 is used for, for example, a culture experiment for confirming the safety of a product in a business place such as a blood center that manufactures blood products. The culture experiment is an experiment of culture of anaerobic bacteria and culture of aerobic bacteria. Therefore, in the culture experiment, two bottles of a first culture bottle 90 (see Fig. 3) for anaerobic culture and a second culture bottle 92 (see Fig. 3) for aerobic culture are used. The collection kit 10 is used to collect a liquid sample (platelet product) and dispense the sample into the two culture bottles 90 and 92 each in a predetermined amount.

Although not particularly limited, an example in which a platelet product is used as a blood product will be described in the present embodiment. As illustrated in Fig. 1, the collection kit 10 includes an inlet tube 12, a collection bag 14, a first sealing member 16, a second sealing member 18, an exhaust valve 20, a first adapter 21a, and a second adapter 21b. In addition, in the collection kit 10, a direction in which the inlet tube 12 and the exhaust valve 20 are located is referred to as an upper side or upward, and a direction in which the first adapter 21a and the second adapter 21b are located is referred to as a lower side or downward based on an arrangement state during use. In addition, a direction perpendicular to the vertical direction and along a plane of the collection bag 14 is referred to as a width direction, and a direction perpendicular to the plane of the collection bag 14 is referred to as a thickness direction.

The inlet tube 12 is, for example, a translucent medical tube made of a thermoplastic resin such as a vinyl chloride resin. The inlet tube 12 can be connected to or separated from another medical tube without exposing the inside thereof to outside air, for example, by an aseptic joining device, a tube sealer, or the like. The inlet tube 12 has an upstream first end 12a and a downstream second end 12b. The first end 12a is welded and sealed in the initial state. The second end 12b is connected to an upper end of the collection bag 14.

The collection bag 14 has two flexible sheets 22 overlapping each other in the thickness direction. The two flexible sheets 22 are welded and joined to each other at a plurality of welding portions 24. Each of the flexible sheets 22 includes a first region 22a for forming one accommodation chamber (a first accommodation chamber 26), a second region 22b for forming the other accommodation chamber (a second accommodation chamber 28), and a connection region 22c connecting the first region 22a and the second region 22b. The first region 22a has a rectangular shape having the same dimensions as the second region 22b in a plan view. The connection region 22c extends in the width direction and is integrally connected to the first region 22a and the second region 22b. Each of the flexible sheets 22 has a pair of cutout portions 22d obtained by cutting out the flexible sheet 22 in a rectangular shape on the upper side and the lower side of the connection region 22c. The connection region 22c is formed between the upper and lower cutout portions 22d, and is formed such that a dimension in the vertical direction is narrower than a dimension in the width direction. Although not particularly limited, the connection region 22c is located at the center in the vertical direction of the first region 22a and the second region 22b.

The collection bag 14 includes the first accommodation chamber 26, the second accommodation chamber 28, and a connection flow path 30 defined by the welding portions 24. The first accommodation chamber 26 is formed in the first regions 22a of the flexible sheets 22. The first accommodation chamber 26 accommodates the platelet product to be introduced into the first culture bottle 90. The first accommodation chamber 26 bulges in the thickness direction of the flexible sheets 22 in the initial state (state at the time of being provided as a product). In the first accommodation chamber 26, one of the flexible sheets 22 and the other flexible sheet 22 are separated from each other in the thickness direction. The first accommodation chamber 26 has a volume for accommodating a predetermined amount (for example, about 8 ml or about 10 ml) of the platelet product.

The collection bag 14 has an inlet port 32 connected to the first accommodation chamber 26 and a first outlet port 34. The inlet port 32 is connected to an upper end of the first accommodation chamber 26 of the collection bag 14. The inlet port 32 is connected to the second end 12b of the inlet tube 12, and allows the inlet tube 12 and the first accommodation chamber 26 to communicate with each other. The first outlet port 34 (outlet port) is connected to a lower end of the first accommodation chamber 26. The first outlet port 34 is connected to the first sealing member 16. The first adapter 21a is connected to the first outlet port 34 via the first sealing member 16.

The second accommodation chamber 28 is formed in the second regions 22b of the flexible sheets 22. The second accommodation chamber 28 accommodates the platelet product to be introduced into the second culture bottle 92. The second accommodation chamber 28 has the same shape as the first accommodation chamber 26 and accommodates the same volume of the platelet product.

The collection bag 14 has a second outlet port 36 and an exhaust port 38 which are connected to the second accommodation chamber 28. The second outlet port 36 (outlet port) is connected to a lower end of the second accommodation chamber 28 of the collection bag 14. The second outlet port 36 is connected to the second sealing member 18. The second adapter 21b is connected to the second outlet port 36 via the second sealing member 18. The exhaust port 38 is connected to an upper end of the second accommodation chamber 28. The exhaust valve 20 is connected to the exhaust port 38.

The connection flow path 30 is a flow path that connects the first accommodation chamber 26 and the second accommodation chamber 28. An upstream end 30a of the connection flow path 30 is connected to an upper portion of the first accommodation chamber 26. The connection flow path 30 extends obliquely upward from the end 30a and is curved at a top portion 30b to extend downward. The connection flow path 30 extends downward to the center in the vertical direction of the collection bag 14 on the downstream side of the top portion 30b, and is bent in the width direction at a bent portion 30c. The connection flow path 30 forms an intermediate portion 30d extending in the width direction in a direction away from the first accommodation chamber 26 on the downstream side of the bent portion 30c. A middle portion of the connection flow path 30 in a flow path direction is located at the center of the intermediate portion 30d in the width direction. The intermediate portion 30d extends along the connection regions 22c of the flexible sheets 22 and extends to the second regions 22b. The connection flow path 30 has a bent portion 30e on the downstream side of the intermediate portion 30d. The connection flow path 30 is bent downward at the bent portion 30e. The connection flow path 30 on the downstream side of the bent portion 30e is turned obliquely upward in a V shape at a folded portion 30f, so that a downstream end 30g located on the downstream side of the folded portion 30f is connected to a lower portion of the second accommodation chamber 28.

The connection flow path 30 bulges in the thickness direction in the initial state. In addition, the intermediate portion 30d of the connection flow path 30 forms a tubular portion 31 having a tubular shape in which a portion passing through the connection region 22c is elongated in the width direction. The tubular portion 31 is sealed by the welding portion 24 whose upper end and lower end are narrow, and the intermediate portion 30d of the connection flow path 30 can be sealed by attaching a clamp 42 through the cutout portions 22d. In addition, the tubular portion 31 can be cut in the sealed state using a tube sealer that cuts a tube while sealing a flow path by heating with high frequency waves or ultrasonic waves.

The first sealing member 16 seals the first outlet port 34 in the initial state to prevent passage of fluid between the first accommodation chamber 26 and the first adapter 21a. The first sealing member 16 is a cylindrical member having a breakable plug therein, and when an internal flow path performs an operation of bending the plug in the initial state, the plug is broken so that the internal flow path can be opened. The second sealing member 18 is a member similar to the first sealing member 16, and seals the second outlet port 36 in the initial state to prevent passage of fluid between the second accommodation chamber 28 and the second adapter 21b.

The exhaust valve 20 communicates with the second accommodation chamber 28 and the first accommodation chamber 26 through the exhaust port 38. The exhaust valve 20 internally has a filter that allows passage of gas and prevents passage of liquid. Although not particularly limited, the exhaust valve 20 may have a hydrophilic filter. When the exhaust valve 20 having the hydrophilic filter comes into contact with the platelet product after the first accommodation chamber 26 and the second accommodation chamber 28 are filled with the platelet product, pores of the filter are clogged by liquid, and thereafter, the exhaust valve 20 prevents the passage of gas and liquid. Such an exhaust valve 20 can prevent backflow of air into the second accommodation chamber 28.

As illustrated in Fig. 3, the first adapter 21a accommodates a neck portion of the first culture bottle 90. The first adapter 21a opens the first sealing member 16 by an internal needle tube penetrating a stopper of the first culture bottle 90, and then causes the first culture bottle 90 and the first accommodation chamber 26 to communicate with each other. The second adapter 21b is a member similar to the first adapter 21a, and opens the second sealing member 18 and then causes the second culture bottle 92 and the second accommodation chamber 28 to communicate with each other.

The collection kit 10 of the present embodiment is configured as described above. The collection kit 10 is used as follows.

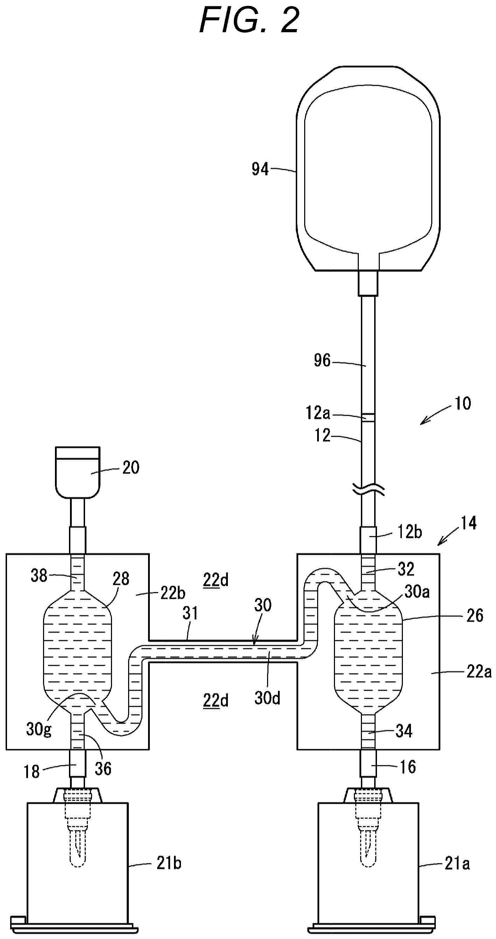

In the present embodiment, a collection method for dispensing the platelet product into the two culture bottles 90 and 92 using the collection kit 10 will be described. First, a platelet bag 94 is joined to the collection kit 10. As illustrated in Fig. 2, the platelet bag 94 is a medical bag accommodating the platelet product. The platelet bag 94 has a tube 96 for connection. The tube 96 is joined to the inlet tube 12. The tube 96 is joined to the inlet tube 12 without coming into contact with outside air using an aseptic joining device.

Next, the platelet product is introduced into the collection bag 14. In this step, in the collection kit 10 and the platelet bag 94, the platelet bag 94 is arranged above the collection bag 14. The first sealing member 16 and the second sealing member 18 of the collection kit 10 are maintained in a closed state.

The platelet product flows out of the platelet bag 94 by gravity and flows into the collection bag 14 through the inlet tube 12. The platelet product flowing into the collection bag 14 flows into the first accommodation chamber 26 through the inlet port 32. The platelet product is stored more and more in the first accommodation chamber 26, and a liquid level of the platelet product in the first accommodation chamber 26 rises. Air inside the first accommodation chamber 26 is pushed out by the platelet product and is discharged from the exhaust valve 20 through the connection flow path 30 and the second accommodation chamber 28.

When the first accommodation chamber 26 is filled with the platelet product, the platelet product flows into the upstream end 30a of the connection flow path 30. Thereafter, the platelet product flows through the connection flow path 30 from upstream to downstream, and flows into the second accommodation chamber 28 from the lower portion of the second accommodation chamber 28. As the inflow of the platelet product proceeds, a liquid level of the platelet product inside the second accommodation chamber 28 rises. Air in the second accommodation chamber 28 is discharged from the exhaust valve 20. The second accommodation chamber 28 is filled with the platelet product. Furthermore, when the exhaust valve 20 comes into contact with the platelet product, the exhaust valve 20 is closed to prevent the inflow of the platelet product. As a result, the introduction of the platelet product into the collection bag 14 is completed. A predetermined amount (for example, about 8 ml) of the platelet product is accommodated in each of the first accommodation chamber 26 and the second accommodation chamber 28.

Thereafter, the platelet bag 94 is separated from the inlet tube 12 by a tube sealer (high frequency or ultrasonic sealer) or the like. The tube sealer separates the inlet tube 12 from the tube 96 of the platelet bag 94, and at the same time, seals the first end 12a of the inlet tube 12 by welding. The separation between the inlet tube 12 and the tube 96 is performed without exposing internal flow paths to the outside air.

The detached platelet bag 94 is stored until a culture test is completed, and then available for use. In addition, the collection kit 10 filled with the platelet product is carried into a clean bench.

Thereafter, as illustrated in Fig. 3, the first culture bottle 90 and the second culture bottle 92 are connected to the first adapter 21a and the second adapter 21b of the collection kit 10, respectively. Thereafter, the tubular portion 31 of the connection flow path 30 is closed by the clamp 42. As the tubular portion 31 is closed, fluid can be prevented from moving between the first accommodation chamber 26 and the second accommodation chamber 28. Note that the tubular portion 31 may be closed while being cut by a tube sealer. In addition, the tubular portion 31 may be closed by a method of welding the two flexible sheets 22 by sealing (also referred to as ultrasonic sealing or high frequency sealing) in which sealing is performed by melting a resin by heating with ultrasonic waves or high frequency waves (electromagnetic waves). In this case, it is not necessary to provide the clamp 42 in a circuit in advance.

Thereafter, an operation of opening the first sealing member 16 is performed, the platelet product in the first accommodation chamber 26 is sucked out due to the negative pressure of the first culture bottle 90, and a predetermined amount of the platelet product is introduced into the first culture bottle 90. In addition, an operation of opening the second sealing member 18 is performed, the platelet product in the second accommodation chamber 28 is sucked out due to the negative pressure of the second culture bottle 92, and a predetermined amount of the platelet product is introduced into the second culture bottle 92.

The first culture bottle 90 is detached from the first adapter 21a, and the second culture bottle 92 is detached from the second adapter 21b. Through the above steps, the collection of the platelet product using the collection kit 10 is completed.

As described above, the collection kit 10 of the present embodiment has a structure in which the collection bag 14 is integrally formed using the two flexible sheets 22, and thus can be manufactured at low cost. In addition, the number of times of joining and separation between the tubes can be minimized, the work efficiency is excellent. Furthermore, since it is not necessary to perform the work of measuring and taking the platelet product by eye according to the scale line, the work becomes easy and the measurement error can be reduced. In addition, the collection kit 10 can reliably separate the first accommodation chamber 26 and the second accommodation chamber 28 by closing the tubular portion 31 of the collection bag 14 with the clamp or the like, and can prevent the platelet product from moving between the first accommodation chamber 26 and the second accommodation chamber 28. Therefore, the collection kit 10 can suppress variations in the amount of the platelet product introduced into each of the first culture bottle 90 and the second culture bottle 92.

### (Second Embodiment)

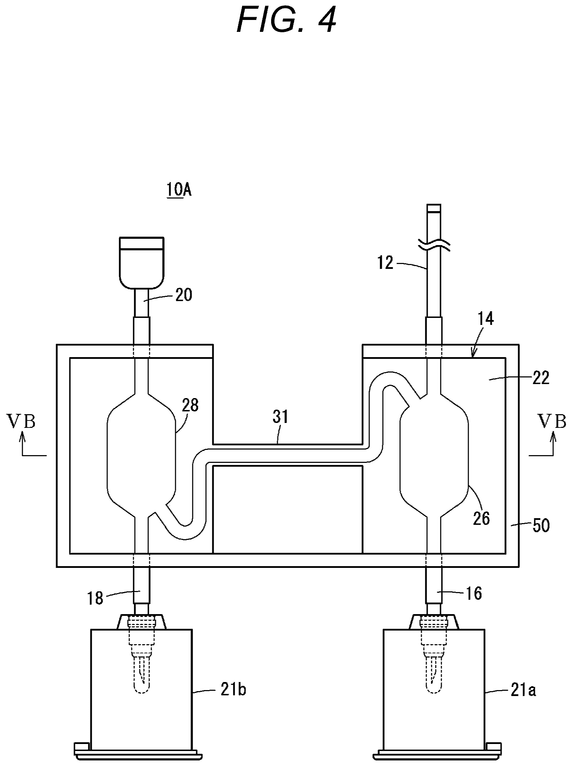

As illustrated in Fig. 4, a collection kit 10A of the present embodiment is different from the collection kit 10 illustrated in Fig. 1 in further including a frame member 50 that holds the collection bag 14. Note that the inlet tube 12, the collection bag 14, the exhaust valve 20, the first adapter 21a, and the second adapter 21b in the collection kit 10A of Fig. 4 are similar to those of the collection kit 10, and thus the detailed description thereof will be omitted.

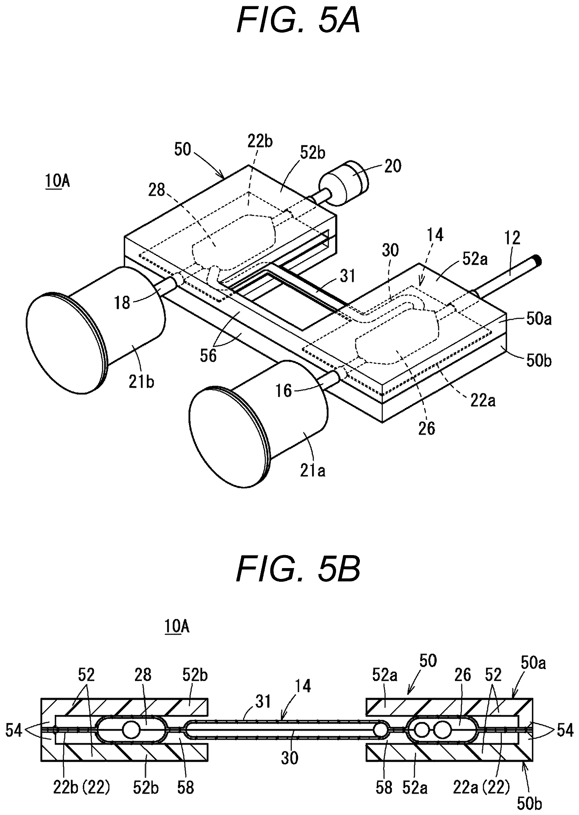

As illustrated in Fig. 5A, the collection kit 10A includes the frame member 50 that holds the collection bag 14. The frame member 50 includes a first frame member 50a and a second frame member 50b. The first frame member 50a is arranged adjacent to one side of the collection bag 14 in the thickness direction. The second frame member 50b is arranged adjacent to the other side of the collection bag 14 in the thickness direction. The frame member 50 has a structure in which the collection bag 14 is sandwiched between the first frame member 50a and the second frame member 50b in the thickness direction.

As illustrated in Figs. 5A and 5B, the first frame member 50a includes an abutment plate 52, a frame body 54, and a connection portion 56. The abutment plate 52 includes a rectangular first flat plate portion 52a formed in a portion corresponding to the first region 22a and a second flat plate portion 52b formed in a portion corresponding to the second region 22b. In the first flat plate portion 52a, the frame body 54 is formed over three sides except for a side facing the center in the width direction. In addition, the frame body 54 is formed over three sides excluding a side facing the center in the width direction in the second flat plate portion 52b. The frame body 54 bulges in the thickness direction with respect to the first flat plate portion 52a and the second flat plate portion 52b. The frame body 54 has a groove to allow passage of a pipe connected to the inlet tube 12, the first sealing member 16, the second sealing member 18, and the exhaust valve 20 in a predetermined part. The first flat plate portion 52a and the second flat plate portion 52b are connected to each other via the connection portion 56. The connection portion 56 is located, for example, at a lower end of the first frame member 50a. The connection portion 56 is away from the tubular portion 31 of the collection bag 14 and is arranged at a position that does not hinder attachment of the clamp 42 or the like to the tubular portion 31.

The first frame member 50a forms recesses 58 in portions surrounded by the frame body 54. The recesses 58 accommodate portions in the thickness direction of the first accommodation chamber 26 and the second accommodation chamber 28, respectively.

The second frame member 50b is formed to be symmetrical with the first frame member 50a in the thickness direction, and includes the abutment plate 52, the frame body 54, and the connection portion 56 similar to those of the first frame member 50a. The second frame member 50b has the recesses 58 in areas surrounded by the frame body 54, and accommodates the other portions in the thickness direction of the first accommodation chamber 26 and the second accommodation chamber 28, respectively.

The abutment plate 52 of the first frame member 50a and the abutment plate 52 of the second frame member 50b abut on each other so as to sandwich the first accommodation chamber 26 and the second accommodation chamber 28 in the thickness direction, and prevent the first accommodation chamber 26 and the second accommodation chamber 28 from expanding (being deformed) in the thickness direction. The abutment plates 52 prevent a volume change due to the expansion of the first accommodation chamber 26 and the second accommodation chamber 28 in the thickness direction, thereby suppressing the variations in the amount of the introduced platelet product.

The frame body 54 of the first frame member 50a and the frame body 54 of the second frame member 50b sandwich and hold a peripheral edge portion of the collection bag 14 in the thickness direction. The frame bodies 54 keep a planar shape of the collection bag 14, thereby improving the handleability of the collection kit 10A. In addition, the frame member 50 having the frame bodies 54 prevents a volume change due to the deformation of the first accommodation chamber 26 and the second accommodation chamber 28, and suppresses the variations in the amount of the introduced platelet product.

Note that a clamp structure that closes the tubular portion 31 may be integrally attached to the frame member 50 in the collection kit 10A of the present embodiment. In this case, work of attaching the clamp 42, prepared separately, to the tubular portion 31 becomes unnecessary, and the operability and handleability are further improved.

The above disclosure is summarized as follows.

According to one aspect, there is provided a collection kit (10) including: an inlet tube (12) to which a medical bag (94) accommodating an object to be collected is connected; a collection bag (14) which is connected to a downstream side of the inlet tube, includes a plurality of accommodation chambers (26, 28) formed by overlapping and welding two flexible sheets (22), and accommodates a predetermined amount of the object to be collected in each of the accommodation chambers; a plurality of outlet ports (34, 36) which are connected to the accommodation chambers, respectively, and allow the object to be collected to flow out from the accommodation chambers; and adapters (21a, 21b) which are connected to downstream sides of the outlet ports, respectively, and connect culture bottles (90, 92) and the outlet ports by the culture bottles being attached to the adapters, respectively, wherein the collection bag includes a connection flow path (30) connecting the accommodation chambers adjacent to each other, and the connection flow path is integrally formed with the accommodation chambers by the two flexible sheets, and has a tubular portion (31) formed in a tubular shape by cutting out the flexible sheets around the tubular portion between the accommodation chambers adjacent to each other.

The collection kit has a structure in which the collection bag is integrally formed using the two flexible sheets, and thus can be manufactured at low cost. In addition, the collection kit can reliably separate the adjacent accommodation chambers by closing the tubular portion of the collection bag with a clamp or the like, and can suppress the variations in the amount of the introduced platelet product.

In the collection kit described above, the plurality of accommodation chambers may include a first accommodation chamber (26) having an upper portion to which the inlet tube is connected, and a second accommodation chamber (28) connected to the first accommodation chamber via the connection flow path, and an exhaust valve (20) that exhausts air inside the first accommodation chamber and the second accommodation chamber may be connected to an upper portion of the second accommodation chamber. This collection kit can smoothly introduce the platelet product by discharging the air inside the first accommodation chamber and the second accommodation chamber through the exhaust valve.

In the collection kit described above, an upstream end (30a) of the connection flow path may be connected to the upper portion of the first accommodation chamber, and a downstream end (30g) of the connection flow path may be connected to a lower portion of the second accommodation chamber. In this collection kit, the platelet product can be smoothly introduced without leaving air in the first accommodation chamber and the second accommodation chamber.

In the collection kit described above, the plurality of outlet ports may include a first outlet port (34) connected to a lower portion of the first accommodation chamber and a second outlet port (36) connected to a lower portion of the second accommodation chamber. In this collection kit, the platelet product in the first accommodation chamber and the platelet product in the second accommodation chamber can be introduced into the culture bottles from the different outlet ports, respectively, and thus, work of transferring the platelet product between different accommodation chambers becomes unnecessary, and the operability is excellent.

The collection kit described above may further include a frame member (50) that holds peripheral edge portions of the flexible sheets and holds the flexible sheets in a planar shape. Since this collection kit can keep the collection bag in a planar shape, work of maintaining the collection bag in a planar shape during use becomes unnecessary, and thus, the handleability is excellent.

In the collection kit described above, the frame member may include: a frame body (54) that holds the peripheral edge portions of the flexible sheets; and an abutment plate (52) that abuts on the accommodation chambers in a thickness direction of the flexible sheets to prevent the accommodation chambers from expanding in the thickness direction. This collection kit can suppress the variations in the amount of the introduced platelet product by suppressing the expansion of the accommodation chambers in the thickness direction.

According to another aspect, there is provided a collection method using the collection kit described above, the collection method including: a step of connecting the medical bag to the inlet tube; a step of introducing the object to be collected into the accommodation chambers of the collection bag through the inlet tube; a step of separating the medical bag from the inlet tube; a step of closing the tubular portion to prevent the object to be collected from moving between the plurality of accommodation chambers; and a step of connecting the culture bottles to the adapters and injecting the object to be collected into the culture bottles. According to this collection method, work efficiency of a culture test of the platelet product can be improved.

In the collection method described above, the tubular portion may be closed by a clamp, ultrasonic sealing, or high frequency sealing in the step of closing the tubular portion to prevent the object to be collected from moving between the plurality of accommodation chambers.

Note that the present invention is not limited to the disclosure, and various configurations can be adopted without departing from the gist of the present invention.

## Claims

1. A collection kit comprising:
an inlet tube to which a medical bag accommodating an object to be collected is connected;
a collection bag which is connected to a downstream side of the inlet tube, includes a plurality of accommodation chambers formed by overlapping and welding two flexible sheets, and accommodates a predetermined amount of the object to be collected in each of the accommodation chambers;
a plurality of outlet ports which are connected to the accommodation chambers, respectively, and allow the object to be collected to flow out from the accommodation chambers; and
adapters which are connected to downstream sides of the outlet ports, respectively, and connect culture bottles and the outlet ports by the culture bottles being attached to the adapters, respectively, wherein
the collection bag includes a connection flow path connecting the accommodation chambers adjacent to each other, and
the connection flow path is integrally formed with the accommodation chambers by the two flexible sheets, and has a tubular portion formed in a tubular shape by cutting out the flexible sheets around the tubular portion between the accommodation chambers adjacent to each other.

2. The collection kit according to claim 1, wherein the plurality of accommodation chambers include a first accommodation chamber having an upper portion to which the inlet tube is connected, and a second accommodation chamber connected to the first accommodation chamber via the connection flow path,
the collection kit further comprising an exhaust valve which is connected to an upper portion of the second accommodation chamber and exhausts air inside the first accommodation chamber and the second accommodation chamber.

3. The collection kit according to claim 2, wherein an upstream end of the connection flow path is connected to the upper portion of the first accommodation chamber, and a downstream end of the connection flow path is connected to a lower portion of the second accommodation chamber.

4. The collection kit according to claim 2 or 3, wherein the plurality of outlet ports include a first outlet port connected to a lower portion of the first accommodation chamber and a second outlet port connected to a lower portion of the second accommodation chamber.

5. The collection kit according to any one of claims 1 to 4, further comprising a frame member which holds peripheral edge portions of the flexible sheets and holds the flexible sheets in a planar shape.

6. The collection kit according to claim 5, wherein the frame member includes: a frame body that holds the peripheral edge portions of the flexible sheets; and an abutment plate that abuts on the accommodation chambers in a thickness direction of the flexible sheets to prevent the accommodation chambers from expanding in the thickness direction.

7. A collection method using the collection kit according to any one of claims 1 to 6, the collection method comprising:
a step of connecting the medical bag to the inlet tube;
a step of introducing the object to be collected into the accommodation chambers of the collection bag through the inlet tube;
a step of separating the medical bag from the inlet tube;
a step of closing the tubular portion to prevent the object to be collected from moving between the plurality of accommodation chambers; and
a step of connecting the culture bottles to the adapters and injecting the object to be collected into the culture bottles.

8. The collection method according to claim 7, wherein
the tubular portion is closed by a clamp, ultrasonic sealing, or high frequency sealing in the step of closing the tubular portion to prevent the object to be collected from moving between the plurality of accommodation chambers.
